# EUROPÄISCHE PATENTSCHRIFT

(11) **EP 4 340 913 B1**
(45) Veröffentlichungstag und Bekanntmachung des Hinweises auf die Patenterteilung: **10.09.2025**
(21) Anmeldenummer: 22728672.1
(22) Anmeldetag: 15.05.2022
(51) Int. Cl.: A61M 13/00, A61B 17/00

(54) **MEDIZINTECHNISCHE PUMPE FÜR DIE ENDOSKOPIE**
MEDICAL PUMP FOR ENDOSCOPY
POMPE MÉDICALE POUR ENDOSCOPIE

(30) Priorität: 17.05.2021 DE 102021002547
(43) Veröffentlichungstag der Anmeldung: 27.03.2024
(73) Patentinhaber: W.O.M. World of Medicine GmbH, 10587 Berlin (DE)
(72) Erfinder: HILDEBRAND, Matthias, 10559 Berlin (DE); KÜRBIS, Stefan, 15831 Mahlow (DE); CHAMBERS, Carl, 13505 Berlin (DE); WINTERBERG, Holger, 13587 Berlin (DE); KRÜGER, Colin M., 13587 Berlin (DE)
(74) Vertreter: Gulde & Partner
(86) Internationale Anmeldenummer: PCT/IB2022/054513
(87) Internationale Veröffentlichungsnummer: WO 2022/243831

(56) Entgegenhaltungen:
- WO-A1-2017/122188
- WO-A1-2018/108200
- WO-A1-2018/173044
- WO-A2-2009/052100
- US-A- 4 971 034
- US-A1- 2019 365 417

## Beschreibung

### Anwendungsgebiet der Erfindung

Es ist bekannt, bei endoskopischen Untersuchungen und insbesondere bei therapeutischen Eingriffen, die jeweilige Körperhöhle durch Fluidzufluss auszudehnen. Im Rahmen der Laparoskopie wird in der Regel in das Abdomen ein Gas (vorzugsweise CO₂) eingebracht und ein Innendruck erzeugt, der höher ist als der Außendruck. Auf diese Weise wird das Abdomen gedehnt, sodass Platz für die Einführung der Operationsgeräte geschaffen wird. Moderne Systeme weisen auch eine Absaugvorrichtung auf, um einerseits sichtbeeinträchtigende Rauchgase schnell entfernen zu können, andererseits aber den Druck in der Körperhöhle während der Operation möglichst konstant zu halten. Diese Systeme sind daher darauf ausgelegt, den Druck in der Körperhöhle möglichst konstant zu halten.

Im Verlauf von Operationen, insbesondere bei langwierigen Operationen, kann es dazu kommen, dass die Sedierung des Patienten nachlässt und dieser bei Reizungen des Gewebes mit Muskelkontraktionen reagiert. Dies führt in der Folge zu einer Erhöhung des Innendrucks von bis zu 60mmHg mit der weiteren Folge, dass das medizintechnische Druckerhaltungssystem die Absaugrate erhöht um den Druck zu verringern. Der Innendruck wird auf diese Weise schnell wieder auf den Sollwert zurückgeführt. Nach Nachlassen der Muskelkontraktionen sinkt der Druck allerdings dann schlagartig weiter ab, was zum Kollaps der Körperhöhle führen kann. Das behandelnde ärztliche Personal muss dann warten, bis sich der Druck wieder aufgebaut hat. Dies verzögert nicht nur den Operationsverlauf, sondern kann auch problematisch sein, z. B wenn im letzten Operationsschritt Gefäße verletzt wurden, so dass Blut austritt, bevor die Gefäße wieder verschlossen werden können.

Zur Überwindung dieses technischen Problems wird die nachfolgend beschriebene medizintechnische Vorrichtung vorgeschlagen, die muskelkontraktionsbedingte Druckerhöhungen erkennt und ein Warnsignal ausgibt.

Eine derartige medizintechnische Vorrichtung vorgeschlagen, die muskelkontraktionsbedingte Druckerhöhungen erkennt, ist aus dem Stand der Technik nicht zu entnehmen. Der Stand der Technik wird u.a. gebildet aus:
WO 2017/122188 A1
US 2019/365417 A1
WO 2018/108200 A1
WO 2018/173044 A1
US 4,971,034 A.

### Grundzüge der Erfindung

Die vorliegende Erfindung betrifft eine Insufflationseinrichtung zur Verwendung in der Medizintechnik, enthaltend
einen Insufflator zum Einbringen von Insufflationsgas in eine Körperhöhle,
eine Regelungseinheit,
eine Insufflationsleitung und eine Desufflationsleitung,
wobei die Desufflationsleitung mit einer Saugpumpe verbunden ist,
wobei die Insufflationsleitung und die Desufflationsleitung je einen Drucksensor und je einen Volumenstromsensor aufweisen,
wobei der Insufflator ein Alarmsignal anzeigt, wenn der Druck in der Körperhöhle durch muskelkontraktionsbedingte Druckspitzen einen Schwellwert über den eingestellten Sollwert überschreitet, ohne dass eine Änderung der Volumenströme den Druckanstieg verursachen könnte.

Die offenbarte medizintechnische Pumpe für die Endoskopie ist in der Lage, muskelkontraktionsbedingte Druckspitzen zu erkennen. Hierzu findet zunächst eine Drucküberwachung in der Körperhöhle statt, wie es im Stand der Technik bereits beschrieben ist. Die Druckdaten werden in der Vorrichtung gespeichert. Weiterhin werden die Betriebsparameter von Zuführpumpe und Absaugpumpe (insbesondere der Gas-Flow) überwacht und ebenfalls gespeichert. Darüber hinaus werden die Betriebsparameter der endoskopischen Instrumente, wie z. B. Endoskope, Katheter und insbesondere HF-Instrumente, z. B. Koagulationselektroden, überwacht. Stellt das System eine Druckerhöhung fest, werden die Betriebsparameter der genannten Geräte überprüft: Stellt das System beispielsweise eine Druckerhöhung fest, nachdem die Zuführpumpe den zufließenden Gasstrom erhöht hat, ohne dass die Absaugpumpe ebenfalls ihren Gasstrom erhöht hat, dann erfolgt die reguläre Druckregelung durch das System, beispielsweise durch Erhöhung der Absaugleistung. Entsprechendes gilt für den Fall, dass bei konstantem Zuführstrom die Absaugleistung vermindert wurde.

Eine Druckerhöhung könnte beispielsweise durch eine Okklusion des Absaugtrokars bewirkt werden. Diese wäre allerdings dadurch zu identifizieren, dass der Gasfluss in der Absaugleitung absinkt.

Stellt das System hingegen fest, dass eine Druckerhöhung auftritt, ohne dass eine Änderung der Gasflussparameter erfolgt ist, dann davon ausgegangen werden, dass die Druckerhöhung durch Muskelkontraktionen ausgelöst wurde. In diesem Fall wird ein Warnsignal ausgelöst. Das Warnsignal kann optischer oder akustischer Natur sein, beispielsweise eine Meldung auf dem Display, wie z. B. "Relaxierung prüfen". Das behandelnde Personal kann dann gegebenenfalls die Sedierung anpassen. Das behandelnde Personal wird natürlich prüfen, ob es einen äußeren Auslöser für Druckerhöhung gibt, z. B. weil der Bauch manuell belastet wurde.

Ein offenbarter Insufflator enthält zunächst einen üblichen Anschluss an eine Druckgasflasche mit einem Gas, welches für medizinische Zwecke geeignet ist, z. B. Kohlendioxid. Das Gas wird über ein Proportionalventil und einen sterilen Schlauch einem Trokar zugeführt. Innerhalb des Insufflators wird sowohl der Druck, als auch der Gasfluss (Flow) der Leitung gemessen. Optional ist noch ein Filter enthalten, der Partikel zurückhält.

Ein zweiter Trokar wird an eine in den Insufflator integrierte Saugpumpe (Desufflationspumpe) angeschlossen. Dabei wird optional wiederum ein Filter zwischengeschaltet, der Partikel, Tröpfchen und/oder giftige Gase absorbieren kann. Das abgepumpte Desufflationsgas kann dann in die Atmosphäre entlassen werden. Auch die Saugpumpe kann geregelt werden und enthält eine Messung des Gasflusses (Flow) der Absaugleitung. Der Insufflator wird über eine Schalteinheit gesteuert, die sowohl den Fluss des zugeführten Gases, wie auch den Fluss des abgesaugten Gases steuern kann.

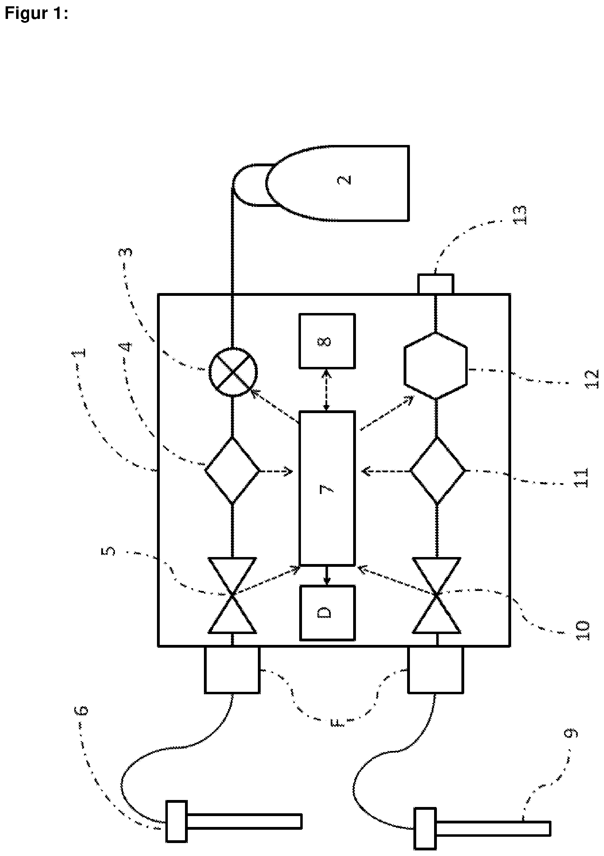

Wie in Figur 1 dargestellt, wird der Insufflator unter Zuhilfenahme zweier Schläuche mit dem Patienten verbunden. Der erste Schlauch wird zur Insufflation verwendet.

Gas wird im Rahmen der Operation dem Patienten zugeführt, um den Druck im Abdomen aufbauen zu können. Weiterhin wird die abdominale Druckmessung über diese Leitung durchgeführt. Über den zweiten Schlauch wird die Absaugpumpe mit dem Patienten verbunden werden, um beispielsweise eine Rauchgasabsaugung zu realisieren.

Als Absaugpumpen kommen elektronisch geregelte Pumpen in Frage, wie sie beispielsweise in der Vorrichtung gemäß DE 102013016063 oder ähnlichen Druckschriften beschrieben sind.

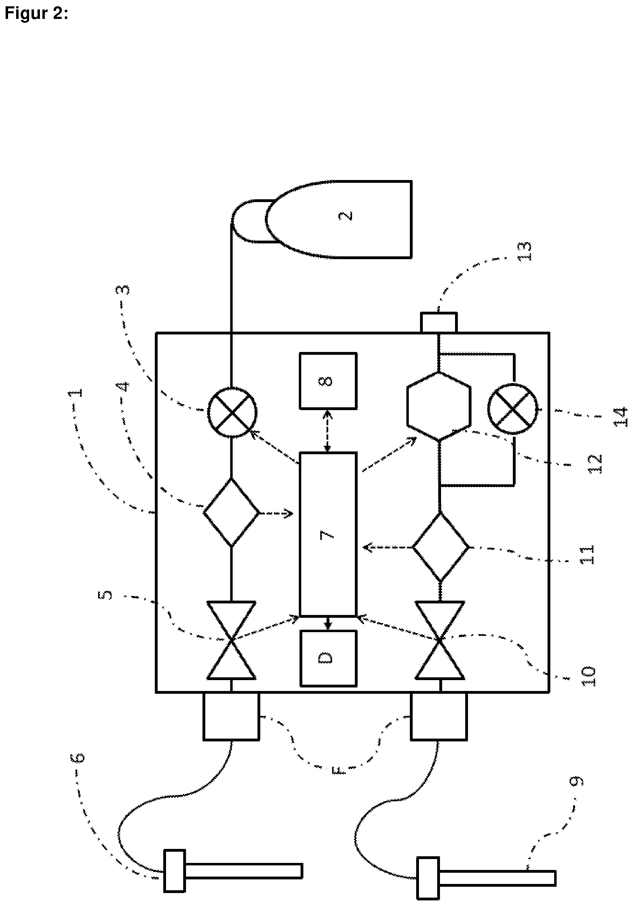

Alternativ kann beispielsweise die Saugpumpe über ein ByPass-Ventil gesteuert werden (Figur 2). Hierzu kann beispielsweise die Pumpe auf eine bestimmte Leistung eingestellt werden, welche weitestgehend konstant ist, und die Regelung der Leistung erfolgt dann über das ByPass-Ventil.

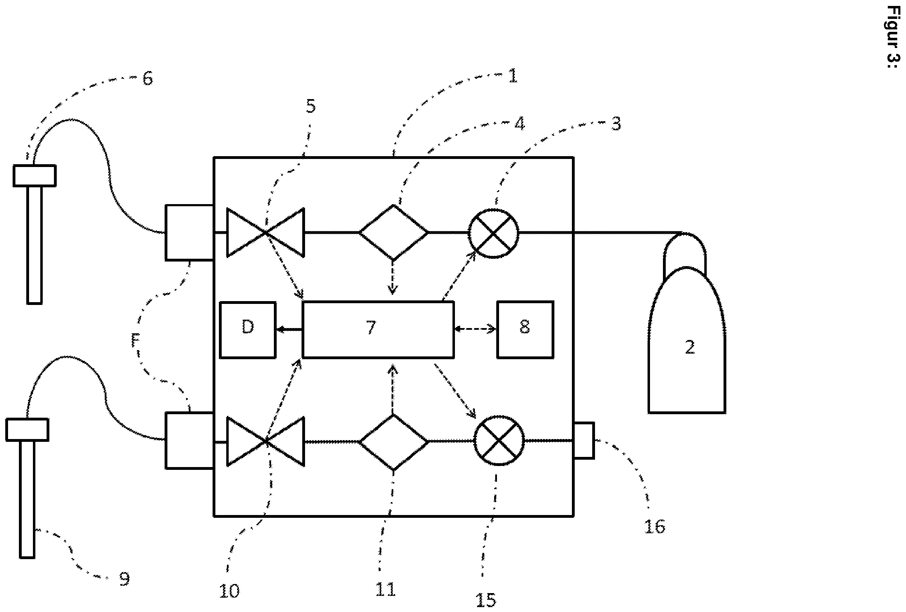

Weiterhin alternativ kann auch ein Steuerungsventil direkt in die Absaugleitung positioniert werden (Figur 3). Auf diese Weise könnten sogar externe Pumpen verwendet werden, beispielsweise die im OP vorhandene Wandabsaugung. Die Regelungseinheit des Insufflators regelt dann die Absaugleistung über das dargestellte Steuerungsventil

Der Insufflator kann weiterhin Sensoren enthalten (optional), die die Betätigung von endoskopischen Instrumenten überwachen können, z. B. von Elektrokoagulationseinrichtungen. Alle aufgenommenen Messdaten werden gespeichert und einer Recheneinheit zugeführt, die die nötige Datenanalyse mittels einer entsprechend ausgelegten Software vornimmt. Bei einem Druckanstieg über einen festgelegten Schwellwert über dem jeweiligen Sollwert, der nicht auf Änderungen der Pumpenparameter zurückzuführen ist, wird der Alarm ausgelöst. Für erfindungsgemäße Vorrichtungen hat sich ein entsprechender Schwellwert von 20 mmHg über dem Sollwert ausgezeichnet bewährt. Abweichende Einstellungen des Schwellwertes auf beispielsweise 10, 30, 40 oder 50mmHg sind möglich.

Weiterhin ist möglich bei einer Druckerhöhung auch die Dauer der Erhöhung zu erfassen. Häufig erfolgen zunächst kurze Kontraktionen (weniger als 10 Sekunden), die zunächst zu entsprechend kurzen Druckerhöhungen (Druckpulsen) über den voreingestellten Schwellwert führen. Das Erkennen mindestens zweier Druckpulse sollte dann den Alarm auslösen.

Bei einer längeren muskelkontraktionsbedingter Druckerhöhung (mehr als 15 - 20 Sekunden) kann die Drucksteuerung des Gerätes nicht nur Alarm auslösen, sondern auch den Druck bis zur Krampflösung verringern. Hierzu ist empfehlenswert den Druck nicht komplett bis zum Sollwert zurückzuregeln, sondern einen leichten Überdruck zu belassen. Wenn der Schwellwert von 20mmHg über dem Sollwert erreicht ist, kann der Druck temporär auf 10mmHg über dem Sollwert eingeregelt werden. Ein solcher temporärer Überdruck beschleunigt den Wiederaufbau des Druckes, insbesondere bei größeren Körperhöhlen (z.B. dem Abdomen).

Weiterhin optional ist es möglich, die Dynamik des Druckanstiegs zur Ernennung einer Muskelkontraktion einzubeziehen. Die hier ursächlichen Muskelkontraktionen führen zu einem relativ schnellen Druckanstieg, verglichen mit der regulären Druckerhöhung durch Änderung der Insufflations- oder Desufflationsparameter. Die Auswertung der Geschwindigkeit des Druckanstiegs erlaubt daher eine genauere Zuordnung der Gründe des Druckanstiegs.

Figur 1 zeigt eine Ausführungsform eines Insufflators. Der Insufflator (1) ist an eine Gasquelle (2), z.B. in Form einer CO₂-Gasflasche angeschlossen. Über ein Proportionalventil (3), einen Drucksensor (4), einen Volumenstromsensor (5) und einen Filter (F) dem Insufflationstrokar (6) zugeführt. Der Desufflationstrokar (9) ist über einen Schlauch mit dem Insufflator verbunden, wobei der Gasflusszunächst wieder über einen Filter (F) einen Volumenstromsensor (10) einem Drucksensor (11) zu einer Saugpumpe (12) führt. Der Ausgang der Saugpumpe führt zu einem Geräteauslass (13). Der Geräteauslass (13) kann selbstverständlich mit einem zusätzlichen Filter ausgestattet sein. Die Messdaten der Drucksensoren (4, 11) sowie der Volumenstromsensoren (5,10) werden an die Recheneinheit (7) mit angeschlossenem Speicher (8) übermitteilt. Die Recheneinheit (7) steuert das Proportionalventil (3) sowie die Saugpumpe (12). Wie der Fachmann erkennen wird, können die Positionen der Drucksensoren und der Volumenstromsensoren auch anders lokalisiert sein: So ist es selbstverständlich möglich, dass der Insufflationsstrom zunächst durch den Volumenstromsensor (5) und dann durch den Drucksensor (4) geführt wird. Ähnlich ist es möglich, dass der Volumenstromsensor (10) der Desufflationsleitung in Strömungsrichtung erst nach der Saugpumpe (12) liegt. In jedem Fall wird bei einer festgestellten muskelkontraktionsbedingten Druckerhöhung um mehr als 20mmHg ein Signal auf dem Display (D) ausgegeben. Das Signal kann auch alternativ oder ergänzend akustisch ausgegeben werden.

Figur 2 zeigt eine Vorrichtung, bei der die Saugpumpe (12) kontinuierlich läuft und die Absaugleistung durch ein By-Pass-Ventil (14) gesteuert wird. Das By-Pass-Ventil (14) wird ebenfalls über die Recheneinheit (7) gesteuert (in der Figur 2 nicht dargestellt).

Figur 3 zeigt eine weitere Variante der Vorrichtung. Hierbei wird statt einer geräteinternen Saugpumpe ein Anschluss (16) für eine externe Pumpe vorgesehen. Viele Krankenhäuser sind mit entsprechenden Pumpen ausgestattet, die für den vorgesehenen Einsatz eines Insufflators verwendet werden können. In diesem Fall wird lediglich ein Steuerungsventil (15) benötigt, um die Saugrate der externen Pumpe (nicht dargestellt) zu regulieren.

Die Einzelkomponenten der Vorrichtung sind größtenteils bereits aus früheren Druckschriften bekannt wie z.B. US 6299592, US5411474, WO1996001132A1, WO 2011041387A1, US 5800381, DE 4219859B4, DE 10 2013 016 063 A1. In diesen Druckschriften sind auch relevante Betriebsverfahren offenbart. Als Regelungseinheit dient ein entsprechend programmierter Microcomputer mit dazugehörigem Speicher sowie Ein- und Ausgabevorrichtungen. Volumenstromsensoren sind bereits aus anderen medizintechnischen Vorrichtungen bekannt (z.B. im Rahmen von Beatmungsvorrichtungen), so dass sie an dieser Stelle nicht weiter erörtert werden müssen.

Die Erfindung wird durch die beigefügten Ansprüche definiert.

## Patentansprüche

1. Insufflationseinrichtung zur Verwendung in der Medizintechnik enthaltend einen Insufflator (1) zum Einbringen von Insufflationsgas in eine Körperhöhle, eine Regelungseinheit (7),
eine Insufflationsleitung und eine Desufflationsleitung,
wobei die Desufflationsleitung mit einer Saugpumpe (12) verbunden ist,
wobei die Insufflationsleitung und die Desufflationsleitung je einen Drucksensor (4,11) und je einen Volumenstromsensor (5,10) aufweisen,
**dadurch gekennzeichnet, dass**
der Insufflator ein Alarmsignal anzeigt, wenn der Druck in der Körperhöhle durch muskelkontraktionsbedingte Druckspitzen einen Schwellwert über den eingestellten Sollwert überschreitet, ohne dass eine Änderung der Volumenströme den Druckanstieg verursachen könnte.

2. Insufflationseinrichtung zur Verwendung in der Medizintechnik gemäß Anspruch 1, wobei der Schwellwert 10, 20, 30, 40 oder 50 mmHg über dem Sollwert ist.

3. Insufflationseinrichtung zur Verwendung in der Medizintechnik gemäß Anspruch 1, wobei das Alarmsignal angezeigt wird, wenn mindestens zwei Druckspitzen über den Schwellwert von jeweils weniger als 10 Sekunden erkannt werden.

4. Insufflationseinrichtung zur Verwendung in der Medizintechnik gemäß Anspruch 1, wobei bei einer Druckspitze von mehr als 15-20 Sekunden über einen Schwellwert von 20mmHg über dem Sollwert ohne dass eine Änderung der Volumenströme den Druckanstieg verursachen könnte, der Alarm ausgelöst wird und der Insufflator temporär auf einen Druck von 10mmHg über dem Sollwert eingeregelt wird.

5. Insufflationseinrichtung zur Verwendung in der Medizintechnik gemäß Anspruch 1-4, wobei der Insufflator bei der Messung des Druckes in der Körperhöhle die Dynamik des Druckanstiegs auswertet.

## Claims

1. An insufflation device for use in medical technology, including an insufflator (1) for introducing insufflation gas into a body cavity,
a control unit (7),
an insufflation line and a desufflation line,
wherein the desufflation line is connected to a suction pump (12),
wherein the insufflation line and the desufflation line each have a pressure sensor (4, 11) and a flow rate sensor (5, 10),
**characterized in that**
the insufflator displays an alarm signal if the pressure in the body cavity exceeds a threshold value above the set target value due to pressure spikes caused by muscle contractions, without it being possible for a change in the flow rate to cause the increase in pressure.

2. The insufflation device for use in medical technology according to Claim 1, wherein the threshold value is 10, 20, 30, 40 or 50 mmHg above the target value.

3. The insufflation device for use in medical technology according to Claim 1, wherein the alarm signal is displayed if at least two pressure spikes above the threshold value, each lasting less than 10 seconds, are detected.

4. The insufflation device for use in medical technology according to Claim 1, wherein, in the case of a pressure spike above the threshold of 20 mmHg above the target value, lasting more than 15-20 seconds, without it being possible for a change in the flow rate to cause the increase in pressure, the alarm is triggered and the insufflator is temporarily adjusted to a pressure of 10 mmHg above the target value.

5. The insufflation device for use in medical technology according to Claims 1-4,
wherein the insufflator evaluates the dynamics of the increase in pressure when the pressure in the body cavity is measured.

## Revendications

1. Dispositif d'insufflation pour utilisation dans la technologie médicale, comportant un insufflateur (1) pour l'introduction de gaz d'insufflation dans une cavité corporelle, une unité de contrôle (7),
une ligne d'insufflation et une ligne de désufflation,
la ligne de désufflation étant reliée à une pompe d'aspiration (12),
la conduite d'insufflation et la conduite de désufflation présentant chacune un capteur de pression (4, 11) et un capteur de débit volumétrique (5, 10),
**caractérisé en ce que**
l'insufflateur affiche un signal d'alarme lorsque la pression dans la cavité corporelle dépasse une valeur seuil au-dessus de la valeur de consigne réglée en raison de pics de pression dus à la contraction musculaire, sans qu'une modification des débits volumétriques puisse provoquer l'augmentation de pression.

2. Dispositif d'insufflation pour utilisation dans la technologie médicale selon la revendication 1, dans lequel la valeur seuil est de 10, 20, 30, 40 ou 50 mmHg au-dessus de la valeur de consigne.

3. Dispositif d'insufflation pour utilisation dans la technologie médicale selon la revendication 1, dans lequel le signal d'alarme est affiché lorsqu'au moins deux pics de pression supérieurs à la valeur seuil de moins de 10 secondes chacun sont détectés.

4. Dispositif d'insufflation pour utilisation dans la technologie médicale selon la revendication 1, dans lequel, en cas de pic de pression de plus de 15-20 secondes au-dessus d'une valeur seuil de 20 mmHg au-dessus de la valeur de consigne sans qu'une modification des débits volumétriques puisse provoquer l'augmentation de pression, l'alarme est déclenchée et l'insufflateur est temporairement réglé à une pression de 10 mmHg au-dessus de la valeur de consigne.

5. Dispositif d'insufflation pour utilisation dans la technologie médicale selon les revendications 1 à 4, dans lequel l'insufflateur évalue la dynamique de l'augmentation de pression lors de la mesure de la pression dans la cavité corporelle.
